# EUROPEAN PATENT APPLICATION

(11) **EP 4 732 895 A1**
(43) Date of publication of application: **29.04.2026**
(21) Application number: 24830785.2
(22) Date of filing: 26.06.2024
(51) Int. Cl.: A61N 5/10

(54) **ADJUSTMENT DEVICE FOR RADIOTHERAPY, ADJUSTMENT METHOD, AND CARRYING SYSTEM**

(30) Priority: 27.06.2023 CN 202310768161; 27.06.2023 CN 202310768156
(71) Applicant: Neuboron Therapy System Ltd., Xiamen, Fujian 361026 (CN)
(72) Inventor: SONG, Chen-ming, Nanjing, Jiangsu 211112 (CN); LIU, Yuan-hao, Nanjing, Jiangsu 211112 (CN); GONG, Qiu-ping, Nanjing, Jiangsu 211112 (CN)
(74) Representative: Gunzelmann, Rainer
(86) International application number: PCT/CN2024/101478
(87) International publication number: WO 2025/002146

(57) **Abstract**

The present application relates to an adjustment device for radiotherapy, an adjustment method and a carrying system. The adjustment device for radiotherapy includes a first adjustment unit and a second adjustment unit, the first adjustment unit being configured to adjust an object to move along a first degree of freedom, and the second adjustment unit being linked to the first adjustment unit and being configured to adjust the object to move along a second degree of freedom, where the first adjustment unit, when working, can drive the second adjustment unit to move along the first degree of freedom. The object is moved along the first degree of freedom by the first adjustment unit and the object is moved along the second degree of freedom by the second adjustment unit, thereby adjusting the posture of an irradiated body to an appropriate treatment position, and increasing the body comfort of the irradiated body during treatment. Compared with conventional manual adjustment means, the first adjustment unit and the second adjustment unit can more accurately adjust the position of the irradiated body, to make the irradiation treatment more accurate, reduce the damage to normal cells and tissues of the irradiated body and ensure the treatment effect for the irradiated body.

## Description

### Technical Field

The present application relates to the technical field of radiotherapy, and in particular to an adjustment device for radiotherapy, an adjustment method, and a carrying system.

### Background

With the development of atomic science, radiotherapy such as cobalt-60, linear accelerators, and electron beams have become one of the principal means for cancer treatment. However, conventional photon or electron therapy is limited by the physical conditions of reflected rays themselves and may cause damages to a large number of normal tissues in a beam path while killing tumor cells. Furthermore, due to differences in the sensitivity of the tumor cells to radioactive rays, the efficacy of conventional radiotherapy is often unsatisfactory for malignant tumors with higher radioresistance.

To reduce radiation damages to normal tissues around a tumor, targeted therapy from chemotherapy has been applied to radiotherapy. For the tumor cells with high radioresistance, currently, radiation sources with high relative biological effectiveness, such as proton therapy, heavy ion therapy, and neutron capture therapy, are actively developed. The neutron capture therapy combines both of the concepts mentioned above. For example, boron neutron capture therapy provides a superior cancer treatment option over conventional radioactive rays by means of the specific accumulation of boron-containing drugs in tumor cells in combination with precise boron beam irradiation.

Radiotherapy apparatuses are mainly used for radiotherapy of malignant tumors, with an aim to maximize the irradiation treatment of a target area while avoiding excessive exposure of surrounding normal tissues to the irradiation. In existing radiotherapy apparatuses, different irradiated bodies usually have different sizes, and a single irradiated body is usually treated at a plurality of body postures, so a carrying device is required to support the irradiated body and position the irradiated body in a preset spatial position. However, during the positioning of the irradiated body in the preset spatial position by the carrying device, how to accurately adjust the posture of the irradiated body, shorten the preparation time for positioning a patient, simplify the preparation and improve the accuracy of irradiation treatment is a problem that a skilled in the art needs to address. Also, during the positioning of the irradiated body in the preset spatial position by the carrying device, how to quickly and accurately adjust the posture of the irradiated body and how to quickly and accurately position the irradiated body in the preset spatial position, and how to avoid aging of the apparatus due to exposure to particle radiation are also problems that a person skilled in the art needs to address.

### Summary

In view of this, it is necessary to provide an adjustment device for radiotherapy, an adjustment method and a carrying system, which are intended to solve the problems of how to quickly, smoothly and accurately adjust the posture of an irradiated body, shorten the preparation time for positioning a patient, simplify the preparation and improve the accuracy of irradiation treatment, how to quickly and accurately position the irradiated body in a preset spatial position, and how to avoid aging of an apparatus due to exposure to radiation.

A first aspect of the present application provides an adjustment device for radiotherapy, including a first adjustment unit and a second adjustment unit, the first adjustment unit being configured to adjust an object to move along a first degree of freedom, and the second adjustment unit being linked to the first adjustment unit and being configured to adjust the object to move along a second degree of freedom, where the first adjustment unit, when working, can drive the second adjustment unit to move along the first degree of freedom.

In the above adjustment device for radiotherapy, the object is moved along the first degree of freedom by the first adjustment unit and the object is moved along the second degree of freedom by the second adjustment unit, thereby adjusting the posture of an irradiated body to an appropriate treatment position, and increasing the body comfort of the irradiated body during treatment. Compared with conventional manual adjustment means, the first adjustment unit and the second adjustment unit of the present application can adjust the position of the irradiated body more accurately, to make the irradiation treatment more accurate, reduce the damage to normal cells and tissues of the irradiated body and ensure the treatment effect for the irradiated body. In addition, the adjustment device of the present application has a compact structure, is easy to operate, and can simplify the preparation before irradiation treatment and reduce the preparation time for patient positioning.

In one embodiment, the first adjustment unit includes a first adjustment member, a first transmission member, and a first support member, the first adjustment member being configured to be connected to and drive the first transmission member to move, the first support member being configured to support the first adjustment member, and the first transmission member being connected to the second adjustment unit.

In one embodiment, the adjustment device further includes a third adjustment unit, the third adjustment unit being configured to adjust the object to move along a third degree of freedom and being linked to the second adjustment unit, and the second adjustment unit, when working, being capable of driving the third adjustment unit to move along the second degree of freedom.

In one embodiment, the second adjustment unit includes a second adjustment member, a second transmission member, a fixed member, and a second support member, the second adjustment member being configured to be connected to and drive the second transmission member to move, the second transmission member being connected to the fixed member, the second support member being configured to support the fixed member, and the second transmission member being connected to the third adjustment unit.

In one embodiment, the third adjustment unit includes a third adjustment member, a swinging member, a third support member, and a first swinging guiding member, the third adjustment member being configured to be connected to and drive the swinging member to move, the third support member being configured to support the third adjustment member, the first swinging guiding member being disposed on the third support member, and the first swinging guiding member being configured to guide a movement path of the swinging member.

In one embodiment, the swinging member is movable relative to the third adjustment member, the first swinging guiding member is configured as an arc such that the swinging member is adjustable relative to a direction of movement of the third adjustment member along the first swinging guiding member.

In one embodiment, a limiting portion is provided on the third adjustment member, an adjustment location for movement of the limiting portion is provided on the swinging member, and when the third adjustment member drives the swinging member to move, the swinging member swings relative to the first swinging guiding member, and the limiting portion moves within the adjustment location.

In one embodiment, the third adjustment unit further includes a rotating member, the object is rotatably connected to the third support member by means of the rotating member, and when the swinging member drives the object to move, the object rotates about an axis of the rotating member.

In one embodiment, the third adjustment unit further includes a second swinging guiding member for guiding the movement of the object, the second swinging guiding member being configured as an arc, and the second swinging guiding member being disposed concentrically with the first swinging guiding member.

In one embodiment, the adjustment device further includes a fourth adjustment unit configured to adjust the object to move at least along a fourth degree of freedom, the fourth adjustment unit being linked to the third adjustment unit, and the third adjustment unit, when working, being capable of driving the fourth adjustment unit to move along the third degree of freedom.

In one embodiment, the fourth adjustment unit includes a fourth adjustment member, a fourth support member, a third transmission member, and a lifting member, the fourth support member being configured to support the fourth adjustment member, the fourth adjustment member being connected to the third transmission member, the third transmission member being connected to the lifting member, the fourth adjustment member driving the third transmission member to move in a first direction, and the third transmission member driving the lifting member to move in a second direction perpendicular to the first direction.

In one embodiment, the fourth adjustment unit further includes a lifting guiding member configured to guide a movement of the lifting member.

In one embodiment, a casing is provided outside at least the first adjustment unit and the second adjustment unit, the casing including at least a first housing and a second housing, and when the first adjustment unit and/or the second adjustment unit move, the first housing and the second housing adjust shapes in a direction of the first degree of freedom and/or the second degree of freedom with the movement of the first adjustment unit and/or the second adjustment unit.

A second aspect of the present application provides an adjustment method for an adjustment device for radiotherapy including a first adjustment unit and a second adjustment unit, the method including: the first adjustment unit, when working, driving the second adjustment unit to move.

In one embodiment, driving the second adjustment unit to move includes: driving the second adjustment unit to move along a first degree of freedom; where the second adjustment unit moves at least along the first degree of freedom.

In one embodiment, the adjustment device further includes a third adjustment unit, and the method further includes: the second adjustment unit, when working, driving the third adjustment unit to move.

In one embodiment, driving the third adjustment unit to move includes: driving the third adjustment unit to move along a second degree of freedom; where the third adjustment unit moves at least along the second degree of freedom.

In one embodiment, the adjustment device further includes a fourth adjustment unit, and the method further includes: the third adjustment unit, when working, driving the fourth adjustment unit to move.

In one embodiment, driving the fourth adjustment unit to move includes: driving the fourth adjustment unit to move along a third degree of freedom; where the fourth adjustment unit moves at least along the third degree of freedom.

A third aspect of the present application provides a carrying system for an adjustment device for radiotherapy, the carrying system including a carrier, a first adjustment unit, and a second adjustment unit; where the first adjustment unit is configured to adjust an irradiated body to move along a first degree of freedom; and the second adjustment unit is linked to the first adjustment unit and is configured to adjust the irradiated body to move along a second degree of freedom, where the first adjustment unit, when working, can drive the second adjustment unit to move along the first degree of freedom.

A fourth aspect of the present application provides an adjustment device for radiotherapy, the adjustment device including an adjustment assembly and a driving assembly, the adjustment assembly being configured to adjust a spatial position of an object, and the driving assembly being configured to drive the adjustment assembly and being detachably connected to the adjustment assembly.

In the above adjustment device for radiotherapy, the adjustment assembly is driven in an automated manner by the driving assembly to adjust the posture of the irradiated body to the appropriate treatment position, improving the body comfort of the irradiated body during treatment, making the irradiation treatment more accurate, reducing the damage to normal cells and tissues of the irradiated body and ensuring the treatment effect for the irradiated body. When it is necessary to adjust the posture of the irradiated body, the driving assembly is connected to the adjustment assembly, and the driving assembly drives the adjustment assembly to adjust the irradiated body to a position aligned to a beam exit to perform the simulated positioning or irradiation treatment of the irradiated body. When the posture of the irradiated body is adjusted to the preset position, the driving assembly is disengaged from the adjustment assembly, and the driving assembly is moved away when the irradiation treatment is performed, such that the driving assembly can be effectively prevented from being affected by particle radiation, thereby preventing the aging of the apparatus and increasing the service life of the driving assembly.

In one embodiment, the adjustment device further includes a mounting assembly, the mounting assembly being disposed on the adjustment assembly and the driving assembly for detachably connecting and fixing the adjustment assembly to the driving assembly.

In one embodiment, the mounting assembly includes a first mounting member and a second mounting member that matches with the first mounting member, the first mounting member and the second mounting member being mounted in positions correspond to each other, the first mounting member being disposed on the adjustment assembly, and the second mounting member being disposed on the driving assembly. Alternatively, the first mounting member is disposed on the first driving assembly, and the second mounting member is disposed on the adjustment assembly.

In one embodiment, the first mounting member includes a first mounting portion, an operating portion, and an engaging portion, the first mounting portion being disposed on the adjustment assembly or the driving assembly, the operating portion being disposed on the first mounting portion, the engaging portion being connected to the operating portion, and the operating portion being configured to drive the engaging portion to engage with the second mounting member.

In one embodiment, the second mounting member includes a second mounting portion and a fixing portion, the second mounting portion being disposed on the driving assembly or the adjustment assembly, the fixing portion being disposed on the second mounting portion, and the fixing portion being configured to fixing the engaging portion.

In one embodiment, the driving assembly includes a power source and an output portion, the power source providing power to the output portion, and the adjustment assembly is provided with an input portion that matches with the output portion in terms of shape, the output portion being detachably connected to the input portion to drive the adjustment assembly for adjustment.

In one embodiment, the driving assembly includes a first driving member and a second driving member linked to the first driving member, the second driving member moves along a first path when the first driving member works, and the second driving member moves along a second path when the second driving member works.

In one embodiment, the first driving member includes a first driving portion, a transmission portion, and a moving portion, the first driving portion being connected to the transmission portion, the moving portion being movably connected to the transmission portion, the first driving portion driving the moving portion to move, and the moving portion being connected to the second driving member.

In one embodiment, the first driving member further includes a transmitting portion configured to transmit power from the first driving portion to the transmission portion, the driving portion and the transmission portion being disposed side by side.

In one embodiment, the second driving member includes a second driving portion and a support portion for supporting the second driving portion, the support portion being connected to the moving portion.

In one embodiment, the second driving member further includes a guiding portion disposed on the support portion, the guiding portion being configured to guide the movement of the second driving portion.

In one embodiment, the driving assembly further includes a third driving member connected to the second driving member, the second driving member and the third driving member move simultaneously along the second path when the second driving member works.

A fifth aspect of the present application provides an adjustment method for an adjustment device for radiotherapy, the adjustment method including the following steps:
connecting and fixing a driving assembly to an adjustment assembly from a disconnected state by the mounting assembly;
interfacing an output portion of the driving assembly with an input portion of the adjustment assembly;
adjusting an object to a preset spatial position by driving the adjustment assembly by the driving assembly; and
disengaging the driving assembly from the adjustment assembly by the mounting assembly.

In the above adjustment method for an adjustment device for radiotherapy, the driving assembly is detachably connected to the adjustment assembly by the mounting assembly, that is, when it is necessary to adjust the posture of the irradiated body, the driving assembly is connected to the adjustment assembly by the mounting assembly to adjust the posture of the irradiated body such that an irradiated site of the irradiated body is aligned to the beam exit. When the posture of the irradiated body is adjusted to the preset spatial position, the driving assembly is disengaged from the adjustment assembly by the mounting assembly, and the driving assembly is then moved away to be prevented from being affected by radiation, thereby increasing the service life of the driving assembly.

In one embodiment, the output portion of the driving assembly includes a first output portion, a second output portion, and a third output portion, and interfacing the output portion of the driving assembly with the input portion of the adjustment assembly includes: adjusting the first output portion to move along the first path and adjusting the second output portion to move along the second path such that the first output portion, the second output portion and the third output portion are interfaced with the input portion.

A sixth aspect of the present application provides a carrying system for radiotherapy, including a carrier, an adjustment assembly, a driving assembly, and a mounting assembly, the carrier being configured to carry an irradiated body, the adjustment assembly being configured to adjust a spatial position of the carrier, the driving assembly being configured to drive the adjustment assembly to adjust the carrier to a preset spatial position, and the mounting assembly being disposed on the adjustment assembly and the driving assembly for detachably connecting the adjustment assembly and the driving assembly.

In the above carrying system for radiotherapy, the adjustment device can more accurately adjust a position of the irradiated body, making the irradiation treatment more accurate, reducing the damage to normal cells and tissues of the irradiated body and ensuring the treatment effect for the irradiated body. Also, the driving assembly is detachably connected to the adjustment assembly by the mounting assembly, the driving assembly can be moved away after the irradiated body is adjusted to the preset spatial position, ensuring that the driving assembly is not affected by particle radiation during irradiation treatment to effectively increase the service life of the driving assembly and save the production cost of the apparatus. An operator can quickly adjust the carrier to the preset spatial position by driving the adjustment assembly by the driving assembly based on different irradiated sites to reduce the preparation time before treatment, such that the irradiated body is aligned to the beam exit, ensuring that the irradiated body undergoes irradiation treatment in a comfortable posture, and ensuring the accuracy of the irradiation treatment.

### Brief Description of the Drawings

FIG. 1 is an overall schematic diagram of a carrier and an adjustment device according to an embodiment of the present invention;
FIG. 2 is a schematic structural diagram of an adjustment assembly according to an embodiment of the present invention;
FIG. 3 is a schematic structural diagram of a first adjustment unit according to an embodiment of the present invention;
FIG. 4 is a schematic structural diagram of a second adjustment unit according to an embodiment of the present invention;
FIG. 5 is a schematic structural diagram of a third adjustment unit according to an embodiment of the present invention;
FIG. 6 is a schematic structural diagram of a first housing and a second housing according to an embodiment of the present invention;
FIG. 7 is a schematic structural diagram of a fourth adjustment unit according to an embodiment of the present invention;
FIG. 8 is a schematic structural diagram of the first housing and the second housing mounted between the carrier and the third adjustment unit according to an embodiment of the present invention;
FIG. 9 is a schematic structural diagram of a first driving assembly according to an embodiment of the present invention;
FIG. 10 is a schematic structural diagram of the first driving assembly of FIG. 8 with the housings removed according to an embodiment;
FIG. 11 is a schematic structural diagram of a first driving member according to an embodiment of the present invention;
FIG. 12 is a schematic diagram of a first moving portion connected to a fourth support portion according to an embodiment of the present invention;
FIG. 13 is a schematic structural diagram of a second driving member according to an embodiment of the present invention;
FIG. 14 is a schematic structural diagram of a third driving member according to an embodiment of the present invention;
FIG. 15 is a schematic structural diagram of a mounting assembly according to an embodiment of the present invention; and
FIG. 16 is a schematic structural diagram of a third moving member according to an embodiment of the present invention.

In the figures, carrier 1, first support portion 101, second support portion 102, adjustment device 2, adjustment assembly 10, first adjustment unit 11, first adjustment member 111, first manipulating portion 1111, first adjustment portion 1112, first transmission member 112, first support member 113, first guiding member 114, first slider 1141, first guide rail 1142, first mounting base 115, second adjustment unit 12, second adjustment member 121, second manipulating portion 1211, second adjustment portion 1212, first gear 1213, second transmission member 122, second gear 1221, fixed member 123, second support member 124, second guiding member 125, second slider 1251, second guide rail 1252, second mounting base 126, third mounting base 127, third adjustment unit 13, third adjustment member 131, third manipulating portion 1311, third adjustment portion 1312, first transmission portion 1313, limiting portion 1314, swinging member 132, adjustment location 1321, third slider 1322, third support member 133, first swinging guiding member 134, third guiding member 135, second swinging guiding member 136, fourth slider 1361, fourth guide rail 1362, rotating member 137, connecting member 138, fourth mounting base 139, first housing 16, first side plate 161, first plate portion 1611, second plate portion 1612, third plate portion 1613, first flat plate 162, second flat plate 163, third flat plate 164, second housing 17, third side plate 171, fifth side plate 172, fourth flat plate 173, fourth adjustment unit 14, fourth adjustment member 141, fourth manipulating portion 1411, fourth adjustment portion 1412, fourth support member 142, third transmission member 143, lifting member 144, first lifting portion 1441, second lifting portion 1442, lifting guiding member 145, fifth support member 146, fifth mounting base 147, sixth mounting base 148, seventh mounting base 149, eighth mounting base 150, third housing 151, fourth housing 152, positioning hole 153, moving assembly 20, push-pull member 21, first moving member 22, driving assembly 30, first driving assembly 31, first driving member 311, first driving portion 3111, first transmitting portion 3112, first transmission wheel 3112a, second driving wheel 3112b, transmitting belt 3112c, second transmission portion 3113, first output portion 3114, first moving portion 3115, eighth mounting base 3116, second driving member 312, second driving portion 3121, second output portion 3122, third support portion 3123, fourth support portion 3124, first guiding portion 3125, fifth slider 3125a, fifth guide rail 3125b, second guiding portion 3126, ninth mounting base 3127, third driving member 313, third driving portion 3131, second transmitting portion 3132, third transmission portion 3133, third output portion 3134, second moving portion 3135, tenth mounting base 3136, eleventh mounting base 3137, limiting member 314, inductive switch 3141, first limiting shaft 3142, second limiting shaft 3143, base 315, fifth housing 316, power source 317, positioning member 318, third moving member 319, connecting block 3191, seventh slider 3192, seventh guide rail 3193, pull rod 3194, twelfth mounting base 3195, limiting block 3196, positioning block 3197, return member 3198, second moving member 320, mounting assembly 40, first mounting member 41, first mounting portion 411, operating portion 412, engaging portion 413, second mounting member 42, second mounting portion 421, fixing portion 422.

### Detailed Description of Preferred Embodiments

In order to make the above objectives, features and advantages of the present application more clearly understood, the particular embodiments of the present application will be described below in detail with reference to the accompanying drawings. In the following description, many specific details are set forth in order to provide thorough understanding of the present application. However, the present application can be implemented in numerous other ways that are different from those described herein, a person skilled in the art can make similar improvements without departing from the connotation of the present application, and therefore the present application is not limited to the particular embodiments disclosed below.

In the description of the present application, it should be understood that the orientation or position relationship indicated by the terms "central", "longitudinal", "transverse", "length", "width", "thickness", "upper", "lower", "front"; "rear", "left", "right", "vertical", "horizontal", "top", "bottom", "inner", "outer", "clockwise", "counterclockwise", "axial", "radial", "circumferential", etc. are based on the orientation or position relationship as shown in the accompanying drawings and are merely intended to facilitate and simplify the description of the present application, rather than indicating or implying that the device or element considered must have a particular orientation or be constructed and operated in a particular orientation, and therefore not to be construed as limiting the present application.

In addition, the terms "first" and "second" are merely for illustrative purposes and are not to be construed as indicating or implying relative importance or to implicitly indicate the number of technical features indicated. Therefore, the features defined with "first" and "second" may explicitly or implicitly include at least one of the features. In the description of the present application, the phrase "a plurality of" means at least two, such as two, three, etc., unless otherwise specifically defined.

In the present application, unless explicitly specified or defined otherwise, the terms such as "mount", "connect", "connected" and "fix" should be interpreted in a broad sense, for example, may be a fixed connection, a detachable connection, or integration; or may refer to mechanical connection or electrical connection, may be a direct connection or an indirect connection via an intermediate medium, or may be communication between interiors of two elements or interaction between the two elements, unless otherwise specifically defined. For those of ordinary skill in the art, the specific meanings of the aforementioned terms in the present application can be understood according to specific conditions.

In the present application, unless otherwise explicitly specified and defined, the first feature being "upper" or "lower" the second feature may refer to the first feature being in direct contact with the second feature, or the first feature being brought into contact with the second feature via an intermediate medium. Moreover, the expression of the first feature being "over", "above" and "on top of" the second feature may be the case that the first feature is directly above or obliquely above the second feature, or only means that the level of the first feature is higher than the second feature. The expression of the first feature being "underneath", "below" and "beneath" the second feature may be the case that the first feature is directly below or obliquely below the second feature, or only means that the level of the first feature is lower than the second feature.

It should be noted that when an element is referred to as being "fixed to" or "disposed on" a further element, it may be directly on the further element, or there may be an intermediate element. When an element is considered to be "connected" to a further element, the element may be directly connected to the further element or an intermediate element may exist simultaneously. The terms "vertical", "horizontal", "upper", "lower", "left", "right" and similar expressions used herein are merely for the purpose of illustration and do not represent any unique implementation.

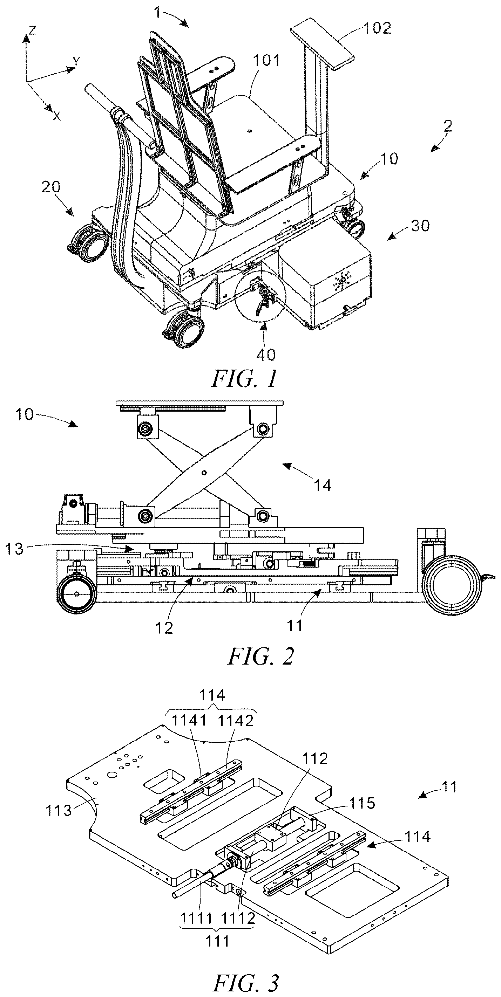

An embodiment of the present application provides a carrying system for radiotherapy. In this embodiment, the carrying system is preferably applied to a radiotherapy apparatus, especially a boron neutron therapy apparatus. The radiotherapy apparatus comprises a beam generation device, a preparation room, a treatment room, a carrier 1, and an adjustment device 2. When the radiotherapy apparatus is the boron neutron therapy apparatus, the beam generation device may be a neutron generation device for generating a beam, the treatment room is used for radioactive irradiation treatment of an irradiated body, and the preparation room can be used to simulate the positioning of the irradiated body or make other preparations before radiation irradiation. Referring to FIG. 1, the carrier 1 is configured to carry an irradiated body. The adjustment device 2 is configured to adjust a posture of the irradiated body in the preparation room and the treatment room to quickly adjust the irradiated body to a preset spatial position.

The adjustment device 2 includes an adjustment assembly 10, a moving assembly 20, a driving assembly 30, and a mounting assembly 40. The adjustment assembly 10 is connected to the carrier 1 for adjusting a spatial position of the carrier 1 to precisely align the irradiated body to the beam. The moving assembly 20 is connected to the adjustment assembly 10 for moving the adjustment assembly 10 and the carrier 1. An output end of the driving assembly 30 is connected to an input end of the adjustment assembly 10 for driving the adjustment assembly 10 to make adjustments such that the carrier 1 is quickly and accurately adjusted to the preset spatial position, thereby saving the preparation time before irradiation treatment and avoiding exposure to a particle irradiation dose for a prolonged period of time. The mounting assembly 40 is configured to connect or separate the adjustment assembly 10 from the driving assembly 30 such that a detachable connection is formed between the driving assembly 30 and the adjustment assembly 10. When it is necessary to adjust the posture of the irradiated body, the driving assembly 30 is connected to the adjustment assembly 10 by the mounting assembly 40, and the driving assembly 30 drives the adjustment assembly 10 to adjust the irradiated body to the preset spatial position for simulated positioning or irradiation treatment of the irradiated body. When the posture of the irradiated body is adjusted to the preset spatial position, the mounting assembly 40 can be released to separate the driving assembly 30 from the adjustment assembly 10, and the driving assembly 30 is moved away when the irradiation treatment is performed, such that mechanical and/or electronic parts inside the driving assembly 30 can be effectively prevented from being affected by particle radiation, thereby increasing the service life of the driving assembly 30.

In other embodiments, the mounting assembly 40 described above may not be provided, as long as a detachable connection between the driving assembly 30 and the adjustment assembly 10 is enabled, and the driving assembly 30 can be removably moved away and can be prevented from being affected by the particle radiation.

Preferably, a neutron beam N generated by the neutron generation device sequentially passes through a beam shaping assembly and a collimator and impinges on the irradiated body on the carrier 1. The beam shaping assembly can adjust the beam quality of the neutron beam N generated by the neutron generation device. The collimator is generally used to converge the neutron beam N to give the neutron beam N high targeting performance during treatment. It may be understood that the present application may also have no collimator, that is, the beam exits the beam shaping assembly and directly impinges on the irradiated body on the carrier 1.

A simulated beam exit identical to a beam exit in the treatment room is provided in the preparation room, and a tumor of the irradiated body is accurately located and marked by a CT image in combination with a laser positioning system, saving the preparation time for the positioning of the irradiated body in the treatment room. In addition, the preparation room can be used as a generic term for all facilities in which various steps are performed before treatment and before the positioning is completed, and may include a preparation room for acquiring an image, a preparation room for positioning, a preparation room for supply of medicines, etc. It may be understood that, in other embodiments, the preparation room may be disposed outside a medical facility or inside a medical facility. It may be understood that the preparation room and the treatment room may be disposed in the same building or different buildings of the medical facility.

Generally, a beam exit is provided in the treatment room, and the neutron beam irradiates the irradiated body for treatment through the beam exit.

The carrier 1 includes a first support portion 101 and a second support portion 102. The first support portion 101 and the second support portion 102 are mounted on the adjustment assembly 10, the first support portion 101 is used for carrying the weight of the irradiated body, and the second support portion 102 is used for carrying an affected region of the irradiated body. In particular, the first support portion 101 may be a seat, a treatment plate, a treatment couch, etc., and the second support portion 102 may be a headrest, a head support, an armrest, a leg rest, etc. In this embodiment, preferably, the first support portion 101 is a seat, the second support portion 102 is a headrest, and the second support portion 102 is disposed in front of the first support portion 101. In actual use, the irradiated body is seated on the first support portion 101, and the head of the irradiated body is placed on the second support portion 102.

The preset spatial position of the carrier 1 is a position for the carrier 1 in which the irradiated body is aligned to the laser or beam. In the present application, the spatial position of the carrier 1 is adjusted from a plurality of degrees of freedom, including: a first degree of freedom (such as the degree of freedom in FIG. 1 for reciprocating parallel to an X axis), a second degree of freedom (such as the degree of freedom in FIG. 1 for reciprocating parallel to a Y axis), a third degree of freedom (rotating about a Z axis), and a fourth degree of freedom (such as the degree of freedom in FIG. 1 for reciprocating parallel to the Z axis). With reference to FIG. 1, a direction perpendicular to a carrying surface of the carrier 1 is defined as a vertical direction (a direction parallel to the Z axis shown in FIG. 1), and a direction (a direction parallel to the Y axis shown in FIG. 1) parallel to an emergent direction of the beam is defined as a front-rear direction, a direction orthogonal to the vertical direction and the front-rear direction is defined as a left-right direction (a direction parallel to the X axis shown in FIG. 1). In the descriptions below, the directional terms such as "upper", "lower", "left", "right", "front" and "rear" are used to describe positional relationships between associated elements, making it simple to describe a positional relationships between one element and another element, but these directional descriptions do not impose any restrictive meaning on the technology itself. In particular, an adjustment along the first degree of freedom refers to the movement of the irradiated body in the left-right direction, an adjustment along the second degree of freedom refers to the movement of the irradiated body in the front-rear direction, an adjustment along the third degree of freedom refers to the rotation of the irradiated body about a straight line parallel to the vertical direction as an axis, and an adjustment along the fourth degree of freedom refers to the movement of the irradiated body in the vertical direction.

As shown in FIG. 2, the adjustment assembly 10 includes a first adjustment unit 11, a second adjustment unit 12, a third adjustment unit 13, and a fourth adjustment unit 14. Preferably, an output end of the first adjustment unit 11 is connected to an input end of the second adjustment unit 12 for adjusting the carrier 1 to drive the object/irradiated body to move along the first degree of freedom. Further, an output end of the second adjustment unit 12 is connected to an input end of the third adjustment unit 13 for adjusting the carrier 1 to drive the object/irradiated body to move along the second degree of freedom. Further, an output end of the third adjustment unit 13 is connected to an input end of the fourth adjustment unit 14 for adjusting the carrier 1 to drive the object/irradiated body to move along the third degree of freedom. Further, an output end of the fourth adjustment unit 14 is connected to the carrier for adjusting the carrier 1 to drive the object/irradiated body to move along the fourth degree of freedom. Preferably, in this embodiment, the first adjustment unit 11, the second adjustment unit 12, the third adjustment unit 13 and the fourth adjustment unit 14 are arranged sequentially from bottom to top, and the fourth adjustment unit 14 is located at the uppermost level and is connected to the carrier 1. In terms of the degree of structural integration and the convenience of a driving process, this arrangement has the smallest footprint and the driving process is the most convenient.

Preferably, the linkage between any two adjacent adjustment units is possible, that is, driving a lower adjustment unit can drive an upper adjustment unit and the carrier 1 to move, and the movement of the upper adjustment unit preferably does not drive the lower adjustment unit to move, which facilitates manipulating and adjusting the individual adjustment units. It is readily understood that driving a lower adjustment unit is represented as driving the input end of this adjustment unit to move, rather than driving the entire adjustment unit to moving in a certain direction.

In particular, the first adjustment unit 11 is linked to the second adjustment unit 12, that is, driving the first adjustment unit 11 to move can drive the second adjustment unit 12, the third adjustment unit 13, the fourth adjustment unit 14 and the carrier 1 to move simultaneously along the first degree of freedom. The second adjustment unit 12 is linked to the third adjustment unit 13, that is, driving the second adjustment unit 12 to move can drive the third adjustment unit 13, the fourth adjustment unit 14 and the carrier 1 to move simultaneously along the second degree of freedom. The third adjustment unit 13 is linked to the fourth adjustment unit 14, that is, driving the third adjustment unit 13 to move can drive the fourth adjustment unit 14 and the carrier 1 to rotate simultaneously along the third degree of freedom. The fourth adjustment unit 14 is connected to the carrier 1, that is, driving the fourth adjustment unit 14 to move can drive the carrier 1 the move along the fourth degree of freedom. The adjustment units are arranged and adjusted in such a way that the carrier 1 can be adjusted in terms of four degrees of freedom to adjust the carrier 1 to the preset spatial position for beam irradiation treatment.

In a preferred embodiment, the adjustment between the adjustment units is in a certain order, and driving the second adjustment unit 12 to move does not affect the position of the first adjustment unit 11. Similarly, driving the third adjustment unit 13 to move does not affect the positions of the first adjustment unit 11 and the second adjustment unit 12. Driving the fourth adjustment unit 13 to move does not affect the positions of the first adjustment unit 11, the second adjustment unit 12 and the third adjustment unit 13. This enables accurate adjustment of each degree of freedom one by one as required under the condition of the adjustments of a plurality of degrees of freedom, thereby reducing the impacts from the independent adjustment of each adjustment unit.

In other embodiments, the first adjustment unit 11, the second adjustment unit 12, the third adjustment unit 13 and the fourth adjustment unit 14 may also be arranged in another order, that is, the order of arrangement of these adjustment units is not limited, as long as the linkage effect can be achieved. For example, the second adjustment unit, the first adjustment unit, the fourth adjustment unit and the third adjustment unit can be arranged sequentially from bottom to top. Also, in other embodiments, any two or any three of the first adjustment unit 11, the second adjustment unit 12, the third adjustment units 13 and the fourth adjustment unit 14 can also be selected for combination, and the order arrangement is not limited, as long as the linkage effect can be achieved. The adjustment assembly 10 designed in levels can achieve targeted adjustment of a single level or a plurality of levels by arbitrarily adjusting one or more of the first adjustment unit 11, the second adjustment unit 12, the third adjustment unit 13 and/or the fourth adjustment unit 14, to smoothly and accurately adjust the posture of the irradiated body in a single or a plurality of degrees of freedom, which reduces the time for repeated operations and verification of adjustment, thereby shortening the preparation time for patient positioning, simplify the preparation and improve the accuracy of the irradiation treatment.

Referring to FIG. 3, the first adjustment unit 11 includes a first adjustment member 111, a first transmission member 112, a first support member 113, and a first guiding member 114. The first adjustment member 111 is connected to the first transmission member 112 and is configured to drive the first transmission member 112 to move. The first transmission member 112 is connected to the second adjustment unit 12, and the second adjustment unit 12 is driven by the movement of the first transmission member 112 to move. The first support member 113 is configured to support the first adjustment member 111, or the like. The first guiding member 114 is connected between the first support member 113 and the second adjustment unit 12 for guiding a path in which the second adjustment unit 12 is driven to move.

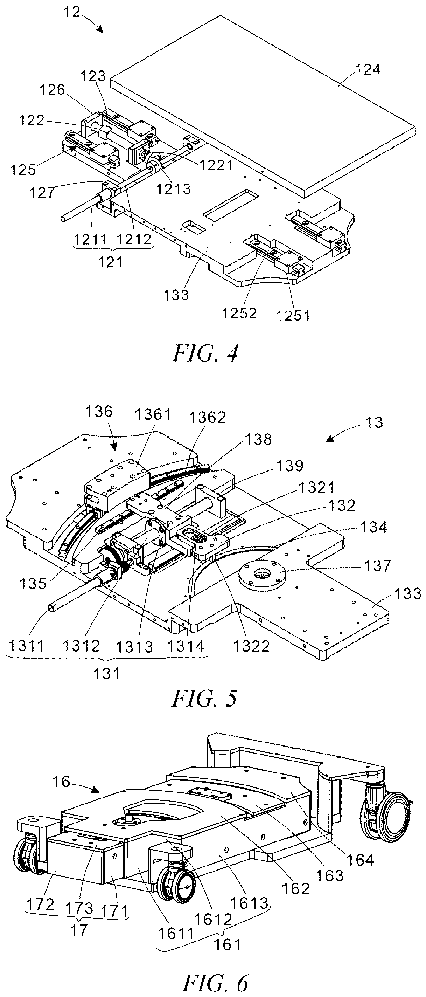

The first adjustment member 111 includes a first manipulating portion 1111 and a first adjustment portion 1112. The first manipulating portion 1111 is configured to control the rotation of the first adjustment portion 1112, and the first adjustment portion 1112 rotates to move the first transmission member 112. In particular, the first manipulating portion 1111 is connected to the first adjustment portion 1112, the first adjustment portion 1112 is mounted to the first support member 113 by means of a first mounting base 115, and the first adjustment portion 1112 is preferably a threaded rod, that is, the first transmission member 112 is threadedly connected to the first adjustment portion 1112. When the first manipulating portion 1111 is rotated, the first transmission member 112 is controllably moved along an axis on which the first adjustment portion 1112 is located by controlling the first adjustment portion 1112, such that a second support member 124 (FIG. 4) is moved in the left-right direction, achieving the movement of the second support member 124 along the first degree of freedom and thus the simultaneous movements of the second adjustment unit 12, the third adjustment unit 13, the fourth adjustment unit 14 and the carrier 1 along the first degree of freedom.

The first guiding member 114 includes a first slider 1141 and a first guide rail 1142. The first slider 1141 is movably connected to the first guide rail 1142. Preferably, two first guiding members 114 are provided. Further, the first guiding members 114 may be disposed on two sides of the first adjustment member 111, respectively, to ensure the balance of support of the second support member 124. Preferably, the first slider 1141 is fixedly connected to the first support member 113, the first guide rail 1142 is fixedly connected to the second support member 124, and the first slider 1141 is slidably connected to the first guide rail 1142, to implement the function of guiding the second support member 124.

Referring to FIG. 4, the second adjustment unit 12 includes a second adjustment member 121, a second transmission member 122, a fixed member 123, the second support member 124, and a second guiding member 125. As in FIG. 4, it may be understood that the presentation of FIG. 4 is not in any limiting sense to the technology itself, and is merely used to describe more specific structures. FIG. 4 shows a view of the second support member 124 and a third support member 133 that are inverted upside down, which can more clearly show the specific structures of the parts of the second adjustment unit 12. During actual use, the second support member 124 is located below the third support member, and the third support member 133 of the third support unit 13 is located above the second support member 124. The second adjustment member 121 is connected to the second transmission member 122 for driving the second transmission member 122 to rotate. The second transmission member 122 is mounted to the third support member 133 of the third adjustment unit 13 by means of a second mounting base 126, to connect the second transmission member 122 and the third adjustment unit 13, and the second transmission member 122 rotates to drive the third adjustment unit 13 to move. The fixed member 123 is fixedly connected to the second support member 124, the second transmission member 122 is preferably a threaded rod, the second transmission member 122 is threadedly connected to the fixed member 123, and the fixed member 123 enables the movement of the second adjustment member 121 and the second transmission member 122 relative to the second support member 124. The second support member 124 is configured to support the fixed member 123, or the like. The second guiding member 125 is connected between the second support member 124 and the third adjustment unit 13 for guiding a movement path of the third adjustment unit 13.

The second adjustment member 121 includes a second manipulating portion 1211 and a second adjustment portion 1212. The second manipulating portion 1211 is configured to control the rotation of the second adjustment portion 1212, and the second adjustment portion 1212 rotates to drive the first transmission member 112 to rotate. The second manipulating portion 1211 is connected to the second adjustment portion 1212, and the second adjustment portion 1212 is mounted to the third support member 133 of the third adjustment unit 13 by means of a third mounting base 127. The second adjustment portion 1212 is preferably a shaft having a certain length. Further, The second adjustment portion 1212 is provided with a first gear 1213. The first gear 1213 meshes with a second gear 1221, and the second gear 1221 is connected to the second transmission member 122. Preferably, the second transmission member 122 is disposed perpendicular to the second adjustment portion 1212, such that the second manipulating portion 1211 and the first manipulating portion 1111 can be arranged on the same side. Advantageously, a second driving member 312 can be integrated with a first driving member 311. When the second manipulating portion 1211 is rotated, the second transmission member 122 is driven by the second adjustment portion 1212, the first gear 1213 and the second gear 1221 to rotate, and since both the fixed member 123 and the second support member 124 are fixed, the second manipulating portion 1211, the second adjustment portion 1212 and the second transmission member 122 are simultaneously moved to drive the third support member 133 to move in the front-rear direction, such that the third support member 133 is moved along the second degree of freedom, and thus the third adjustment unit 13, the fourth adjustment unit 14 and the carrier 1 are moved simultaneously along the second degree of freedom.

The second guiding member 125 includes a second slider 1251 and a second guide rail 1252. The second slider 1251 is movably connected to the second guide rail 1252. At least two second guiding members 125 are provided, and the at least two guiding members may be disposed on two sides of the second adjustment member 121, respectively, to better ensure the balance of the third support member 133. The second slider 1251 is fixedly connected to the second support member 124, the second guide rail 1252 is fixedly connected to the third support member 133, and the second slider 1251 is slidably connected to the second guide rail 1252, to implement the function of guiding the third support member 133.

Referring to FIG. 5, the third adjustment unit 13 includes a third adjustment member 131, a swinging member 132, a third support member 133, a first swinging guiding member 134, a third guiding member 135, a second swinging guiding member 136, and a rotating member 137. The third adjustment member 131 is movably connected to the swinging member 132 for driving the swinging member 132 to move. The swinging member 132 is connected to the fourth adjustment unit 14 such that the fourth adjustment unit 14 is rotated by the swinging of the swinging member 132. The third support member 133 is configured to support the third adjustment member 131, or the like. The first swinging guiding member 134 is disposed on the third support member 133 and is movably connected to the swinging member 132 for guiding a swinging path of the swinging member 132. A first transmission portion 1313 is connected to a third guiding member 135 by a connecting member 138, and the third guiding member 135 is configured to guide a movement path of the first transmission portion 1313. The second swinging guiding member 136 is connected between the third support member 133 and the fourth adjustment unit 14 for guiding a movement path of the fourth adjustment unit 14. The rotating member 137 is connected between the third support member 133 and the fourth adjustment unit 14, and the rotating member 137 is preferably an annular or circular structure. When the swinging member 132 swings, the rotating member 137 drives the fourth adjustment unit 14 to rotate substantially about a central axis of the rotating member 137. It is readily understood that when the rotating member 137 is another form of a structure, the rotating member 137 drives the fourth adjustment unit 14 to rotate substantially about a further axis. The swinging member 132 is movably connected to the third adjustment member 131 and the first guiding member 114, and the swinging member 132 swings by means of the third adjustment member 131 and the first guiding member 114.

The third adjustment member 131 includes a third manipulating portion 1311, a third adjustment portion 1312, the first transmission portion 1313, and a limiting portion 1314. The third manipulating portion 1311 is configured to control the rotation of the third adjustment portion 1312, the third adjustment portion 1312 rotates to move the first transmission portion 1313, and the first transmission portion 1313 moves to drive the swinging member 132 to move. The limiting portion 1314 is configured to enable the swinging member 132 to be movably connected to the first transmission portion 1313. Preferably, the third manipulating portion 1311 is connected to the third adjustment portion 1312 by means of gear meshing, the third adjustment portion 1312 is mounted to the third support member 133 by means of a fourth mounting base 139, the third adjustment portion 1312 is preferably a threaded rod, and the third adjustment portion 1312 is threadedly connected to the first transmission portion 1313. When the third manipulating portion 1311 is rotated, the first transmission portion 1313 is driven by the third adjustment portion 1312 to move along an axis on which the third adjustment portion 1312 is located, so as to drive the connecting member 138 to move along the axis on which the third adjustment portion is located. The swinging member 132 is connected to a fourth support member 142 of the fourth adjustment unit 14, the sliding of the limiting portion 1314 in an adjustment location 1321 and the guiding of the swinging member 132 by the first swinging guiding member 134 enable the swinging of the swinging member 132 in the first swinging guiding member 134, which in turn drives the fourth support member 142 to rotate, so as to achieve the rotation of the fourth support member 142 along the third degree of freedom and thus the simultaneous rotation of the fourth adjustment unit 14 and the carrier 1 along the third degree of freedom.

It is readily understood that the limiting portion 1314 may be a protrusion, a bump, or the like. The limiting portion 1314 is disposed on the connecting member 138, the first transmission portion 1313 is connected to the connecting member 138, and the connecting member 138 is movably connected to the swinging member 132 by means of the limiting portion 1314. The swinging member 132 is provided with an adjustment location 1321 for movement of the limiting portion 1314. For example, the adjustment location 1321 may be a slot, a hole, or the like that has a certain length. In this embodiment, the adjustment location 1321 is preferably an oblong hole that matches the limiting portion 1314, thereby allowing the limiting portion 1314 to move flexibly within the oblong hole.

Further, the swinging member 132 is movable relative to the third adjustment member 131, and the first swinging guiding member 134 may be configured as an arc. In particular, the first swinging guiding member 134 is an arc-shaped groove, a third slider 1322 is provided at a bottom of the swinging member 132, and the swinging member 132 is moved in the arc-shaped groove by means of the third slider 1322. Preferably, the swinging member 132 may be disposed on a side opposite the arc-shaped opening of the arc-shaped groove. When the first transmission portion 1313 drives the swinging member 132 to move, the swinging member 132 is guided along an arc-shaped path by the first swinging guiding member 134, and in combination with the first transmission portion 1313 driving the swinging portion to move, the swinging member 132 swings along the arc-shaped path.

Further, the third guiding member 135 is a linear guide rail. Two or more third guiding members 135 may be provided and fixed to the third support member 133 such that the two third guiding members 135 are located on two sides of the axis on which the third adjustment portion 1312 is located, respectively. Two ends of the first transmission portion 1313 are movably connected to the two third guiding members 135 by means of the connecting member 138, respectively, for stable and balanced guiding of the first transmission portion 1313.

Further, the second swinging guiding member 136 includes a fourth slider 1361 and a fourth guide rail 1362. The fourth slider 1361 is movably connected to the fourth guide rail 1362. The fourth guide rail 1362 is preferably configured as an arc, and the arc of the fourth guide rail 1362 is disposed concentrically with the arc of the first swinging guiding member 134, for example, with a center of their circle being an intersection between an axis on which the rotating member 137 is located and a plane on which the third support member 133 is located. This can further improve the stability and synchronization of adjustment. The fourth guide rail 1362 is disposed on the third support member 133, the fourth slider 1361 is fixedly connected to the fourth support member 142, and the fourth slider 1361 is slidably connected to the fourth guide rail 1362, thereby further implementing the function of guiding the fourth support member 142.

Further, the rotating member 137 may be a bearing. The rotating member 137 is fixedly connected to the third support member 133 and is rotatably connected to the fourth support member 142 of the fourth adjustment unit 14, such that the fourth adjustment unit 14 can be rotated relative to the third support member 133 by the rotating member 137.

Referring to FIG. 6, a casing is provided outside the first adjustment unit 11, the second adjustment unit 12 and the third adjustment unit 13. Adjustments can be made to the shape of the casing with the movements of the second adjustment unit 12 and the third adjustment unit 13, and the casing is configured to protect the parts inside the first adjustment unit 11, the second adjustment unit 12 and the third adjustment unit 13. Preferably, the casing further includes a first housing 16 and a second housing 17. The first housing 16 is disposed outside the second housing 17. In particular, The first housing 16 is an outer housing, and the second housing 17 is an inner housing. The first housing 16 is connected to the second support member 124, and the first housing 16 can move with the second support member 124. The second housing 17 is connected to the third support member 133, and the second housing 17 can move with the third support member 133. For example, when the first manipulating portion 1111 is rotated, the second support member 124 can move in the left-right direction while the first housing 16 and the second housing 17 move in the left-right direction with the second support member 124. When the second manipulating portion 1211 is rotated, the third support member 133 can move in the front-rear direction while the second housing 17 moves in the front-rear direction along with the third support member 133.

Preferably, the first housing 16 includes a first side plate 161, a second side plate (not shown), a first flat plate 162, a second flat plate 163, and a third flat plate 164. The first side plate 161 and the second side plate are substantially identical in terms of structure, the first side plate 161 and the second side plate are oppositely disposed and connected to two sides of the second support member 124, respectively. The first side plate 161 includes a first plate portion 1611, a second plate portion 1612, and a third plate portion 1613. The first plate portion 1611, the second plate portion 1612 and the third plate portion 1613 may be integrally formed in the same plane or not in the same plane. In the embodiments disclosed herein, two adjacent plate portions of the first plate portion 1611, the second plate portion 1612 and the third plate portion 1613 are connected to each other and are in the form of a bend with a bending angle of 90°. It may be understood that the configurations and shapes of the first plate portion 1611, the second plate portion 1612 and the third plate portion 1613 are not limited as long as they can enclose the sides of the second housing 17 and the second support member 124. The first flat plate 162, the second flat plate 163, the third flat plate 164 are disposed on the top of the third adjustment unit 13, the first flat plate 162, the second flat plate 163, the third flat plate 164 are connected to the first side plate 161 and the second side plate, and the first flat plate 162, the second flat plate 163 and the third flat plate 164 together protect the internal structures of the third adjustment unit 13 and also function to support the fourth adjustment unit 14.

It is readily understood that the first housing 16 is movably disposed outside the second housing 17, the first housing and the second housing enclose the main structures of the first adjustment unit 11, the second adjustment unit 12 and the third adjustment unit 13 in an adjustable manner, and with any adjustment of the first adjustment unit 11, the second adjustment unit 12 and the third adjustment unit 13, the first housing 16 and the second housing 17 can be adaptively adjusted in a coordinated manner for comprehensive enclosure in first, second and third dimensions, i.e., in the right-left, front-rear and rotational directions, improving the overall sealing and safety of the adjustment assembly. The first housing 16 and the second housing 17 may be made of a radiation shielding material, especially a material selected for shielding against neutrons, photons and gamma rays, etc., and the split-type housing can effectively protect the structures and parts of the driving assembly and minimize radiation damage to the internal structures.

Preferably, the second housing 17 includes a third side plate 171, a fourth side plate (not shown), a fifth side plate 172, and a fourth flat plate 173. The third side plate 171 and the fourth side plate are substantially identical in terms of structure and are disposed oppositely and are connected to two sides of the third support member 133, respectively, and the third side plate 171 and the fourth side plate are at least partially located inside of the first side plate 161 and the second side plate. The fifth side plate 172 is connected on two sides to the third side plate 171, the fourth side plate, respectively, and the third side plate 171, the fourth side plate and the fifth side plate 172 together enclose the form of C to enclose some of the parts of the first adjustment unit 11, the second adjustment unit 12 and the third adjustment unit 13 inside. The fourth flat plate 173 is connected to the third side plate 171, the fourth side plate and the fifth side plate 172, and the fourth flat plate 173 is located below the first flat plate 162. When the third support member 133 moves in the front-rear direction, the fourth flat plate 173 can adaptively cover the parts therein to protect the parts from the outside.

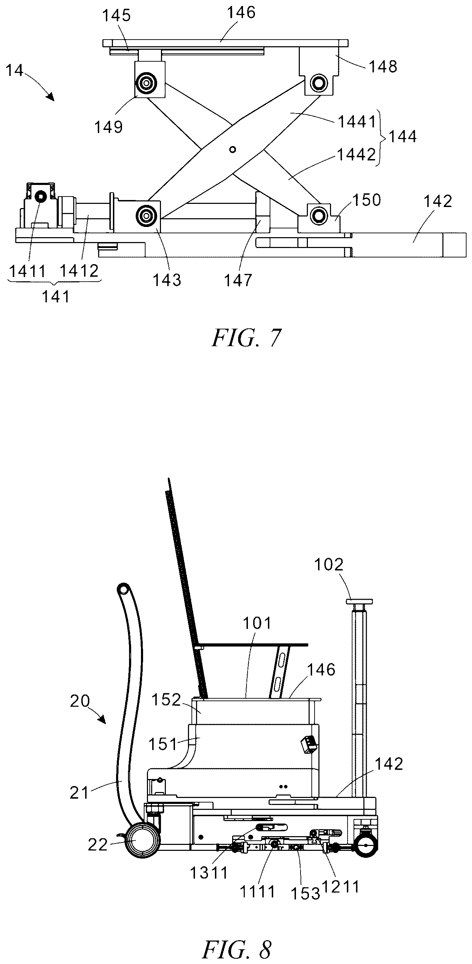

Referring to FIG. 7, the fourth adjustment unit 14 includes a fourth adjustment member 141, a fourth support member 142, a third transmission member 143, a lifting member 144, a lifting guiding member 145, a fifth support member 146, a third housing 151, and a fourth housing 152. The fourth adjustment member 141 is connected to the third transmission member 143 for driving the third transmission member 143 to move in a first direction. The third transmission member 143 is connected to the lifting member 144, and the third transmission member 143 moves to drive the lifting member 144 to move in a second direction perpendicular to the first direction, such as the vertical direction, such that the carrier 1 disposed on the fifth support member 146 moves along the fourth degree of freedom. The fourth support member 142 is configured to support the fourth adjustment member 141, or the like. The lifting guiding member 145 is slidably connected to the lifting member 144 for guiding the lifting member 144. The fifth support member 146 is connected to the lifting guiding member 145 and the carrier 1 for mounting the carrier 1 and the lifting guiding member 145. The third housing 151 and the fourth housing 152 are disposed around a periphery of the lifting member 144 for protecting the fourth adjustment unit 14. It is readily understood that, in other embodiments, the lifting guiding member 145 may also be disposed on the fourth support member 142, i.e., not connected to the fifth support member 146. By means of sliding connection with the lifting member 144, the guiding of the adjustment of the lifting member 144 is also achieved.

Further, the fourth adjustment member 141 includes a fourth manipulating portion 1411 and a fourth adjustment portion 1412. The fourth manipulating portion 1411 is configured to control the movement (such as rotation) of the fourth adjustment portion 1412, and the fourth adjustment portion 1412 rotates to move the third transmission member 143. The first manipulating portion 1111 is connected to the fourth adjustment portion 1412 by means of a reducer, the fourth adjustment portion 1412 is mounted to the fourth support member 142 by means of a fifth mounting base 147, the fourth adjustment portion 1412 is preferably a screw rod, and the third transmission member 143 is threadedly connected to the fourth adjustment portion 1412.

Further, the lifting member 144 includes a first lifting portion 1441 and a second lifting portion 1442. The first lifting portion 1441 and the second lifting portion 1442 are hinged at middle portions. A first end of the first lifting portion 1441 is hinged to a sixth mounting base 148, the sixth mounting base 148 is fixedly connected to the fifth support member 146, and a second end of the first lifting portion 1441 is hinged to the third transmission member 143. Further, a first end of the second lifting portion 1442 is hinged to a seventh mounting base 149, the seventh mounting base 149 is slidably connected to the lifting guiding member 145, a second end of the second lifting portion 1442 is hinged to an eighth mounting base 150, and the eighth mounting base 150 is fixedly connected to the fourth support member 142. When the fourth manipulating portion 1411 is rotated, the third transmission member 143 is driven by the fourth adjustment portion 1412 to move in the first direction, such as moving along an axis on which the fourth adjustment portion 1412 is located, to drive the first lifting portion 1441 and the second lifting portion 1442 to rotate synchronously relative to each other, achieving the movement of the fifth support member 146 in the second direction perpendicular to the first direction, a movement in the vertical direction in this embodiment, and achieving the movement of the carrier 1 along the fourth degree of freedom.

Referring to FIG. 8, the third housing 151 is connected to the fourth support member 142, and the fourth housing 152 is connected to the fifth support member 146. Preferably, in one embodiment, the third housing 151 and the fourth housing 152 are provided in a split-type telescopically adjustable form, where the fourth housing 152 is disposed around the periphery of the lifting member 144, the third housing 151 is disposed around a periphery of the fourth housing 152, and the fourth housing 152 is movable relative to the third housing 151 to adapt to a lifting movement of the lifting member 144.

The third housing 151 and the fourth housing 152 may be provided in an integral form, in addition to in the split-type form. In other embodiments, the fourth housing 152 may not be provided, and only the third housing 151 is provided. For example, one end of the third housing 151 is connected to the fourth support member 142, and the other end of the third housing 151 is connected to the fifth support member 146. In this embodiment, the third housing 151 is telescopically deformable. For example, the first housing 51 may be of a flexible or corrugated structure, and the flexible or corrugated structure of the first housing 151 can be telescoped for shape adjustment with the movement of the lifting member 144, thereby protecting the movement of the lifting member 144. It may be understood that the first housing 151 and the second housing 152 are not limited in terms of form and shape as long as they can adjust their shapes with the movement of the lifting member 144 and can function to protect the lifting member 144.

Further, the moving assembly 20 includes a push-pull member 21 and first moving members 22. The push-pull member 21 is disposed on a side of the adjustment assembly 10 for pushing or pulling the adjustment assembly 10 and the carrier 1, and the push-pull member 21 may be a handle, a hand rail, a push-pull rod, or the like. The first moving members 22 are disposed around a bottom of the adjustment assembly 10 for moving the adjustment assembly 10 and the carrier 1, such as moving the adjustment assembly 10 and the carrier 1 as a whole to the preparation room, the treatment room, or the like, and the first moving members 22 may be pulleys, casters, rollers, universal wheels, chain wheels, or the like.

Further, At least one of the first manipulating portion 1111, the second manipulating portion 1211, the third manipulating portion 1311 and the fourth manipulating portion 1411 of the first adjustment unit 11, the second adjustment unit 12, the third adjustment unit 13 and the fourth adjustment unit 14 may be manually operated, or the first manipulating portion 1111, the second manipulating portion 1211, the third manipulating portion 1311 and the fourth manipulating portion 1411 may be driven by the driving assembly 30. It is readily understood that the driving assembly 30 can adjust the carrier 1 to the preset spatial position more quickly and accurately compared to manual operations.

Further, the driving assembly 30 includes a first driving assembly 31 and/or a second driving assembly (not shown). For example, the first driving assembly 31 is configured to drive the first manipulating portion 1111, the second manipulating portion 1211 and the third manipulating portion 1311 to rotate. The second driving assembly is configured to drive the fourth manipulating portion 1411 to rotate.

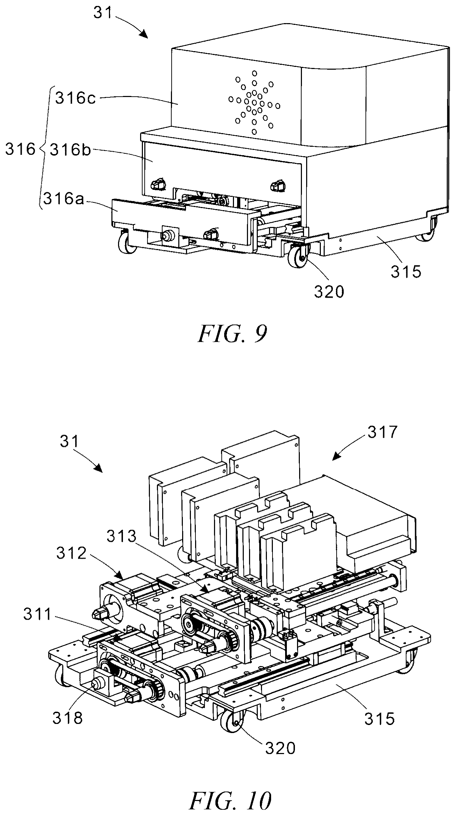

As shown with respect to FIGS. 9, 10 and 14, the first driving assembly 31 includes a first driving member 311, a second driving member 312, a third driving member 313, a limiting member 314, a base 315, a fifth housing 316, a power source 317, and second moving members 320. An output portion of the first driving member 311 is connected to the first manipulating portion 1111 for driving the first manipulating portion 1111 to rotate. An output portion of the second driving member 312 is connected to the second manipulating portion 1211 for driving the second manipulating portion 1211 to rotate. An output portion of the third driving member 313 is connected to the third manipulating portion 1311 for driving the third manipulating portion 1311 to rotate. The first driving member 311, the second driving member 312 and the third driving member 313 are each provided with the limiting member 314 for limiting movement travels of the first adjustment unit 11, the second adjustment unit 12 and the third adjustment unit 13. The base 315 is configured to support the first driving member 311, the second driving member 312, the third driving member 313, etc. The fifth housing 316 is configured to encase the power source 317, the first driving member 311, the second driving member 312 and the third driving member 313, etc. It is readily understood that the power source 317 may include an AC/DC power source, a pneumatic structure, etc. The power source 317 is configured to supply power to the first driving member 311, the second driving member 312 and the third driving member 313. The second moving member 320 is connected to the base 315 for moving the entire first driving assembly 31. Preferably, in this embodiment, the first driving member 311, the second driving member 312 and the third driving member 313 are integrated as one piece by the base 315 and the fifth housing 316, and are externally mounted outside of the adjustment assembly 10. The first driving assembly 31 can be removably moved away to avoid being affected by particle radiation during irradiation treatment, which otherwise causes the activation and loss of the internal parts. The second driving assembly includes a fourth driving member (not shown), an output portion of the fourth driving member is connected to the fourth manipulating portion 1411 for driving the fourth manipulating portion 1411 to rotate.

The fifth housing 316 may be provided in an integral form, or may be designed in a split-type form according to the structure of the driving assembly, such as split into three housings 316a, 316b and 316c. In particular, the housing 316a and the housing 316b are configured to protect the first driving member 311, the second driving member 312 and the third driving member 313, and the housing 316c is configured to protect the power source 317. The split-type structure can be separated and closed during the alignment and adjustment of the driving assembly and the adjustment device. The fifth housing 316 may be made of a radiation shielding material, especially a material selected for shielding against neutrons, photons and gamma rays, etc., and the split-type housing can effectively protect the structures and parts of the driving assembly and minimize radiation damage to the internal structures.

In other embodiments, the fourth driving member may also be integrated into the first driving assembly 31.

Further, the first driving member 311 is in linkage with the second driving member 312, that is, when the first driving member 311 works, the second driving member 312 can be moved along a first path, and when the second driving member 312 works, the second driving member 312 itself can move along a second path. The second driving member 312 is in linkage with the third driving member 313, that is, when the second driving member 312 works, the third driving member 313 can move along the second path with the second driving member 312. The linkage process of the three driving members is described below.

In a preferred embodiment, the second driving member 312, when working, will not affect the position of the first driving member 311. Similarly, the third driving member 313, when working, will not affect the positions of the first driving member 311 and the second driving member 312. In this way, the driving assembly 30 can drive the adjustment assembly 10 exactly one by one as required to achieve the movements of the carrier 1 along a plurality of degrees of freedom.

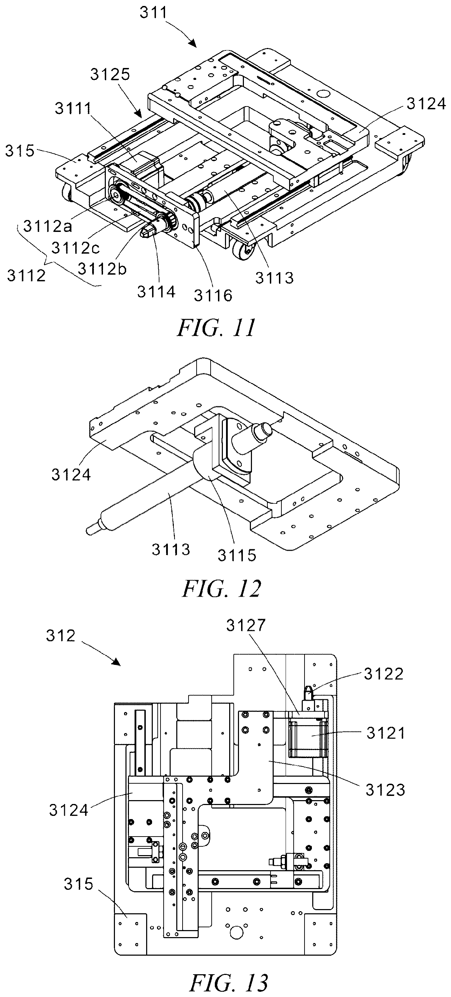

Referring to FIGS. 11 and 12, the first driving member 311 includes a first driving portion 3111, a first transmitting portion 3112, a second transmission portion 3113, a first output portion 3114, and a first moving portion 3115. The first driving portion 3111 is configured to provide a driving force. The first transmitting portion 3112 is connected between the first driving portion 3111 and the second transmission portion 3113, and the first transmitting portion 3112 is configured to transmit the power from the first driving portion 3111 to the second transmission portion 3113. The second transmission portion 3113 is configured to rotate with the first driving portion 3111. The first output portion 3114 is connected to an end of the second transmission portion 3113 for outputting power from the first driving portion 3111 to drive the first manipulating portion 1111 to rotate. The first moving portion 3115 is drivingly connected to the second transmission portion 3113 and is connected to the second driving member 312 for driving the second driving member 312 and the third driving member 313 to move.

Further, the first driving portion 3111 is mounted to the eighth mounting base 3116, and there is a shaft at an end of the first driving portion 3111. The first transmitting portion 3112 includes a first transmission wheel 3112a, a second driving wheel 3112b, and a transmitting belt 3112c. The first transmission wheel 3112a is disposed on the shaft of the first driving portion 3111, the second driving wheel 3112b is disposed at an end of the second transmission portion 3113, and the transmitting belt 3112c meshes with the first transmission wheel 3112a and the second driving wheel 3112b for transmitting the driving force from the first driving portion 3111 to the second transmission portion 3113.

Further, the second transmission portion 3113 and the first driving portion 3111 may be disposed side by side, which saves the footprint and makes the arrangement compact, and an end of the second transmission portion 3113 is also mounted to the eighth mounting base 3116. The second transmission portion 3113 may be a screw rod, and the first moving portion 3115 is threadedly connected to the second transmission portion 3113. Under the actions of the first driving portion 3111 and the first transmitting portion 3112, the second transmission portion 3113 rotates, and the first moving portion 3115 moves along an axis on which the second transmission portion 3113 is located. The first moving portion 3115 is connected to the fourth support portion 3124, and the first moving portion 3115 moves to drive the second driving member 312 and the third driving member 313 to move (described in detail below).

Further, the first output portion 3114 is connected to the end of the second transmission portion 3113, a sleeve for transmitting power is provided at an end of the first manipulating portion 1111 close to the first output portion 3114, and the first output portion 3114 is connected to the first manipulating portion 1111 by means of the sleeve. The first manipulating portion 1111 is rotated by the first output portion 3114 under the action of the first driving portion 3111, achieving the movements of the second adjustment unit 12, the third adjustment unit 13, the fourth adjustment unit 14 and the carrier 1 along the first degree of freedom.

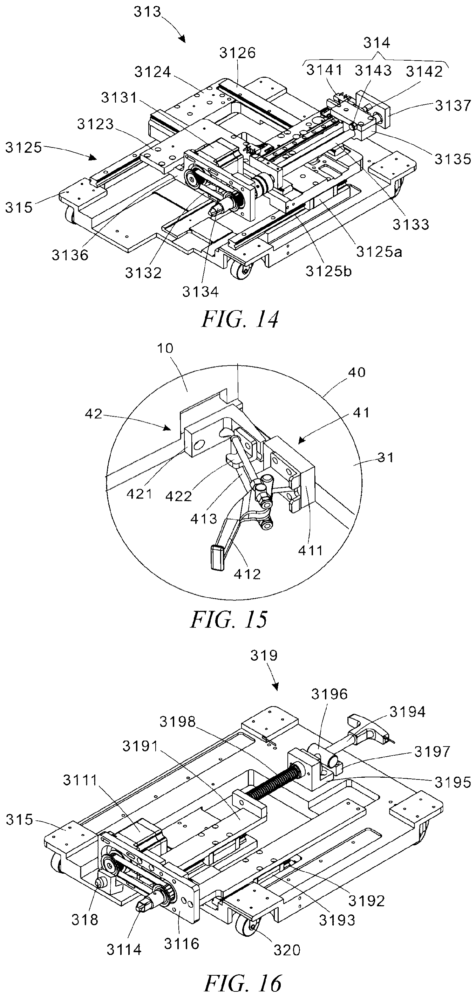

In the embodiments disclosed herein, a lead of the second transmission portion 3113 is preferably the same as a lead of the first adjustment portion 1112. In particular, with respect to FIG. 14, the same threaded rod is preferably used for the second transmission portion 3113 and the first adjustment portion 1112, and the second transmission portion 3113 and the first moving portion 3115 are configured, in combination with the limiting member 314, to be able to limit a movement path of the first transmission member 112. A movement travel of the first moving portion 3115 on the second transmission portion 3113 is the same as a movement travel of the first transmission member 112 on the first adjustment portion 1112. The first moving portion 3115 moving to the end of the second transmission portion 3113 represents that an upper limit of movement of the first transmission member 112 is reached, that is, the second adjustment unit 12 reaches an upper limit of adjustment along the first degree of freedom, at this point the first moving portion 3115 comes into contact with the limiting member 314, and under the action of the limiting member 314, the first driving portion 3111 idles or stops rotating to stop the movement of the second adjustment unit 12, ensuring the safe operation of the entire device within an adjustable range.

Referring to FIGS. 12, 13 and 14, the second driving member 312 includes a second driving portion 3121, a second output portion 3122, a third support portion 3123, a fourth support portion 3124, a first guiding portion 3125, and a second guiding portion. The second driving portion 3121 is configured to provide a driving force. The second output portion 3122 is connected to an output end of the second driving portion 3121 for outputting power from the second driving portion 3121 to drive the second manipulating portion 1211 to rotate. The third support portion 3123 is connected to the second driving portion 3121 for mounting the second driving portion 3121. The fourth support portion 3124 is connected to the first moving portion 3115 for moving with the first moving portion 3115. The first guiding portion 3125 is disposed at the fourth support portion 3124 and is connected between the fourth support portion 3124 and the base 315, and the first guiding portion 3125 is configured to guide the second driving portion 3121 to move along a first path defined by the first guiding portion 3125. The second guiding portion is disposed at the third support portion 3123 and is connected between the third support portion 3123 and the fourth support portion 3124, and the second guiding portion is configured to guide the second driving portion 3121 to move along a second path defined by the second guiding portion.

Further, a sleeve is provided at an end of the second manipulating portion 1211 close to the second output portion 3122, and the second output portion 3122 is connected to the second manipulating portion 1211 by means of the sleeve. The second manipulating portion 1211 is driven by the second output portion 3122 to rotate under the action of the second driving portion 3121, achieving the movements of the third adjustment unit 13, the fourth adjustment unit 14 and the carrier 1 along the second degree of freedom.

Further, the first guiding portion 3125 includes a fifth slider 3125a and a fifth guide rail 3125b. The fifth slider 3125a and the fifth guide rail 3125b may also be disposed in pairs on the base 315. The second guiding portion includes a sixth guide rail 3126. Preferably, the third support portion 3123, the second guiding portion, the fourth support portion 3124 and the first guiding portion 3125 are disposed sequentially from top to bottom on the base 315. The second driving portion 3121 is mounted to the third support portion 3123 by means of a ninth mounting base 3127, the third support portion 3123 is connected at a bottom to a sixth slider (not shown). The sixth slider is slidably connected to the sixth guide rail 3126, and the sixth guide rail 3126 is connected to the fourth support portion 3124. The fourth support portion 3124 is connected at a bottom to the fifth slider 3125a, the fifth slider 3125a is slidably connected to the fifth guide rail 3125b, and the fifth guide rail 3125b is fixedly connected to the base 315. The fourth support portion 3124 is connected at the bottom to the first moving portion 3115 for moving with the first moving portion 3115. Preferably, a straight line on which the fifth guide rail 3125b is located is perpendicular to a straight line on which the sixth guide rail 3126 is located. Referring to the aforementioned setting of the plurality of degrees of freedom in the adjustment assembly 10, in the embodiments disclosed herein, the straight line on which the fifth guide rail 3125b is located is parallel to the first degree of freedom, and the second driving portion 3121 moves by means of the fifth guide rail 3125b, that is, the second driving portion 3121 moves along the first path. The straight line on which the sixth guide rail 3126 is located is parallel to the second degree of freedom, and the second driving portion 3121 moves by means of the sixth guide rail 3126, that is, the second driving portion 3121 moves along the second path. When the first driving portion 3111 drives the first manipulating portion 1111 to rotate, the first transmission member 112 moves along an axis on which the first adjustment portion 1112 is located, to drive the second adjustment unit 12 to move along the first degree of freedom while the first moving portion 3115 also moves along the axis on which the second transmission portion 3113 is located, that is, the first moving portion 3115 moves synchronously with the second adjustment unit 12. Since the first moving portion 3115 is connected to the fourth support portion 3124, the fourth support portion 3124 also moves with the second adjustment unit 12 to enable the movement of the second driving portion 3121 along the first path, such that the second output portion 3122 may not be disengaged from the second manipulating portion 1211, that is to say, the second output portion 3122 may not be disengaged from the sleeve of the second manipulating portion 1211, or otherwise the second manipulating portion 1211 cannot be driven. In particular, the first guiding portion 3125 is configured to guide the fourth support portion 3124 to move along the first path. When the second driving portion 3121 drives the second manipulating portion 1211 to rotate, the second manipulating portion 1211, the second adjustment portion 1212, the second transmission member 122 and the third support member 133 move together along the second degree of freedom while the second driving portion 3121 also moves along the second path, such that the second output portion 3122 may not be disengaged from the second manipulating portion 1211, that is to say, the second output portion 3122 may not be disengaged from the sleeve of the second manipulating portion 1211, or otherwise the second manipulating portion 1211 cannot be driven. In particular, the second guiding portion is configured to guide the second driving portion 3121 and to drive the second output portion 3122 to move along the second path.

Referring to FIG. 14, the third driving member 313 includes a third driving portion 3131, a second transmitting portion 3132, a third transmission portion 3133, a third output portion 3134, and a second moving portion 3135. The third driving portion 3131 is configured to provide a driving force. The second transmitting portion 3132 is connected between the third driving portion 3131 and the third transmission portion 3133 for transmitting power from the third driving portion 3131 to the third transmission portion 3133. The second transmitting portion 3132 of the third driving member 313 is substantially identical to the first transmitting portion 3112 of the first driving member 311 in terms of structure and function, which will not be repeated herein. The third output portion 3134 is connected to an end of the third transmission portion 3133 for outputting power from the third driving portion 3131 to drive the third manipulating portion 1311 to rotate. The third transmission portion 3133 and the second moving portion 3135 are configured, in combination with the limiting member 314, to limit the movement path of the first transmission portion 1313 (described in detail below).

Further, the third driving portion 3131 is mounted to the third support portion 3123 by means of a tenth mounting base 3136, and the third transmission portion 3133 is mounted to the third support portion 3123 by means of an eleventh mounting base 3137. The third output portion 3134 is located at the end of the third transmission portion 3133, and the third output portion 3134 is connected to the third manipulating portion 1311 by means of the sleeve.

Further, when the first driving portion 3111 drives the first manipulating portion 1111 to rotate, the second adjustment unit 12 and the third adjustment unit 13 move along the first degree of freedom while the second driving portion 3121 and the third driving portion 3131 move along the first path by means of the first guiding portion 3125. When the second driving portion 3121 drives the second manipulating portion 1211 to rotate, a part of the second adjustment unit 12 and the third adjustment unit 13 move along the second degree of freedom while the second driving portion 3121 and the third driving portion 3131 move along the second path by means of the second guiding portion 3126. When the third driving portion 3131 drives the third manipulating portion 1311 to rotate, the fourth adjustment unit 14 moves along the third degree of freedom. That is to say, preferably, at least the first output portion is not disengaged from the first manipulating portion. Further, the second output portion is not disengaged from the second manipulating portion, and the third output portion is not disengaged from the third manipulating portion. In this way, during adjustment, the driving assembly 30 can reliably and continuously adjust the adjustment assembly 10 without manually and frequently coupling different output shafts for adjustment, which saves operation time.

Preferably, in the embodiments disclosed herein, a lead of the third transmission portion 3133 is preferably the same as a lead of the third adjustment portion 1312. In particular, the same threaded rod is preferably used for the third transmission portion 3133 and the third adjustment portion 1312, and the second moving portion 3135 is threadedly connected to the third transmission portion 3133. A movement travel of the second moving portion 3135 on the third transmission portion 3133 is the same as a movement travel of the first transmission portion 1313 on the third adjustment portion 1312. The second moving portion 3135 moving to the end of the third transmission portion 3133 represents that an upper limit of movement of the first transmission portion 1313 along the second degree of freedom is reached, that is, the third adjustment unit 13 reaches an upper limit of adjustment, at this point the second moving portion 3135 comes into contact with the limiting member 314, and under the action of the limiting member 314, the third driving portion 3131 idles or stops rotating to stop the movement of the third adjustment unit 13, ensuring the safe operation of the entire device within the adjustable range.

Further, the limiting member 314 includes an inductive switch 3141 and a limiting shaft. Preferably, the inductive switch 3141 as a soft limit and the limiting shaft as a hard limit are provided on each of the first driving member 311, the second driving member 312 and the third driving member 313 to carry out double limit on the movement travels of the first adjustment unit 11, the second adjustment unit 12 and the third adjustment unit 13 such that the adjustment units cannot be further adjusted when they reach the upper limit of adjustment, ensuring safe operation of the device. It may be understood that only the inductive switch 3141 or only the limiting shaft or both the inductive switch and the limit shaft may be provided. In the embodiments disclosed herein, the arrangement scheme of the limiting member 314 is described in connection with the third driving member 313, and the arrangement of the limiting member 314 for the first driving member 311 and the second driving member 312 is substantially identical in terms of method and principle to that of the limiting member 314 for the third driving member 313 and will not be repeated herein.

Further, the inductive switch 3141 is mounted to the third support portion 3123, two inductive switches 3141 can be provided, the two inductive switches 3141 are located on two sides of the eleventh mounting bases 3137, respectively, and correspond to two ends of the third transmission portion 3133, respectively. When the second moving portion 3135 moves along the third transmission portion 3133 to one end of the third transmission portion 3133, the second moving portion 3135 comes into contact with the inductive switch 3141, i.e., indicating that the third adjustment unit 13 reaches the upper limit of adjustment, and at this point the third driving portion 3131 can stop driving by power-off such that the first transmission portion 1313 stops moving.

Further, the limiting shaft includes a first limiting shaft 3142 and a second limiting shaft 3143. The first limiting shaft 3142 is mounted to the eleventh mounting base 3137, and the second limiting shaft 3143 is mounted to the second moving portion 3135. When the second moving portion 3135 moves along the third transmission portion 3133 to one end of the third transmission portion 3133, the second moving portion 3135 abuts against the first limiting shaft 3142 or the second limiting shaft 3143 abuts against the eleventh mounting base 3137, i.e., indicating that the third adjustment unit 13 reaches the upper limit of adjustment, and at this point the third driving portion 3131 idles such that the first transmission portion 1313 stops moving.

Further, the second moving members 320 are disposed around a bottom of the base 315 to facilitate moving the entire first driving assembly 31. The second moving members 320 may be pulleys, casters, rollers, universal wheels, or the like.

Further, the second driving assembly includes a fourth driving member. the fourth driving member is provided separately, and a fourth output portion of the fourth driving member is connected to the fourth manipulating portion 1411 by means of the sleeve.

Referring to FIG. 15, the mounting assembly 40 includes a first mounting member 41 and a second mounting member 42. The first mounting member 41 is preferably detachably connected to the second mounting member 42 for detachable connection and fixation of the adjustment assembly 10 and the first driving assembly 31. The first mounting member 41 matches with the second mounting member 42 and the two are mounted in positions correspond to each other. The first mounting member 41 may be mounted to the adjustment assembly 10, and the second mounting member 42 may be mounted to the first driving assembly 31. Alternatively, the first mounting member 41 may be mounted to the first driving assembly 31, and the second mounting member 42 may be mounted to the adjustment assembly 10. In the embodiments disclosed herein, two first mounting members 41 are provided, the two first mounting members 41 are mounted on two sides of the first driving assembly 31, respectively. Two second mounting members 42 are provided, and the two second mounting members 42 are mounted on two sides of the adjustment assembly 10, respectively.

The second driving assembly may also be mounted by using the mounting assembly 40 of the present application. It may be understood that other mounting means may also be used for mounting.

In the embodiments disclosed herein, the first mounting member 41 includes a first mounting portion 411, an operating portion 412, and an engaging portion 413. The first mounting portion 411 is preferably mounted on a side face of the base 315 of the first driving assembly 31, the operating portion 412 is disposed on the first mounting portion 411, the engaging portion 413 is connected to the operating portion 412, and the operating portion 412 is configured to drive the engaging portion 413 to engage with a fixing portion 422 of the second mounting member 42.

The second mounting member 42 includes a second mounting portion 421 and the fixing portion 422. The second mounting portion 421 is preferably mounted to a side face of the bottom of the adjustment assembly 10, the fixing portion 422 is disposed on the second mounting portion 421, and the fixing portion 422 is configured to fix the engaging portion 413. The engaging portion 413 has two positions, including a clamping position and a released position. In the clamping position, the engaging portion 413 locks the adjustment assembly 10 and the first driving assembly 31. In the released position of the engaging portion 413, the adjustment assembly 10 is disengaged from the first driving assembly 31. The operating portion 412 can operably drive the engaging portion 413 to move between the clamping position and the released position. Preferably, the engaging portion 413 has a loop structure, and the fixing portion 422 is in the form of a hook. When it is necessary to adjust the spatial position of the carrier 1, the first driving assembly 31 is moved to a mounting position of the adjustment assembly 10, the engaging portion 413 is driven by the operating portion 412 to move toward the fixing portion 422 such that the loop structure of the engaging portion 413 passes through the fixing portion 422 in the form of a hook, and the operating portion 412 is then moved away from the engaging portion 413 and locked. The fixing portion 422 can stop the engaging portion 413 from being disengaged therefrom to maintain the engaging portion 413 in the clamping position, and at this point the engaging portion 413 is in the clamping position to fix the first driving assembly 31 relative to the adjustment assembly 10, achieving the connection and fixing of the first driving assembly 31 and the adjustment assembly 10. When the carrier 1 is adjusted to the preset spatial position, the operating portion 412 is driven toward the fixing portion 422, the engaging portion 413 is separated from the fixing portion 422, and at this point the engaging portion 413 is in the released position such that the first driving assembly 31 can be moved relative to the adjustment assembly 10, achieving the disengagement of the first driving assembly 31 from the adjustment assembly 10.

An embodiment of the present application provides an adjustment method for an adjustment device. The method includes the following steps.

In step S100, the first driving assembly 31 and/or the second driving assembly (not shown) are connected and fixed to the adjustment assembly 10 from a disengaged state by the mounting assembly 40.

In actual operation, step S100 includes the following steps.

In step S101, the carrier 1 is moved to the preparation room.

In step S102, the first driving assembly 31 is positioned in a mounting position.

With respect FIGS. 8 and 16, the base 315 of the first driving assembly 31 is further provided with a positioning member 318, and a positioning hole 153 is correspondingly provided at the bottom of the adjustment assembly 10. The positioning hole 153 matches with the positioning member 318 for pre-positioning the first driving assembly 31 in the mounting position, facilitating subsequent positioning by the mounting assembly 40. The second moving members 320 are provided between the first driving member 311 and the base 315, and a third moving member 319 allows the first driving member 311 to move relative to the base 315 for manual position adjustment.

The third moving member 319 includes a connecting block 3191, a seventh slider 3192, a seventh guide rail 3193, a pull rod 3194, a twelfth mounting base 3195, a limiting block 3196, a positioning block 3197, and a return member 3198. The connecting block 3191 is connected to the eighth mounting base 3116, the connecting block 3191 is connected at a bottom to the seventh slider 3192, the seventh slider 3192 is slidably connected to the seventh guide rail 3193, and the seventh guide rail 3193 is connected to the base 315, such that the first driving member 311 can be operably moved relative to the base 315. An axis of the seventh guide rail 3193 is preferably parallel to the first degree of freedom such that the first driving member 311 drives the first output portion 3114 to move along the first path. The connecting block 3191 is connected to one end of the pull rod 3194, the pull rod 3194 passes through the twelfth mounting base 3195, the twelfth mounting base 3195 is fixed to the base 315, and the limiting block 3196 is rotatably connected to the pull rod 3194, the positioning block 3197 is disposed at a distance from the twelfth mounting base 3195, and the return member 3198 is sleeved on the pull rod 3194 and is located between the connecting block 3191 and the twelfth mounting base 3195. It readily understand that the return member 3198 can be selected from a spring, a torsion spring, a biasing structure, etc. The pull rod 3194 is configured to pull the connecting block 3191 to move the first driving member 311 along the first path, the limiting block 3196 is then rotated to abut against the positioning block 3197, and at this point the first driving member 311 is further away from the adjustment assembly 10 than the positioning member 318. When the positioning member 318 matches with the positioning hole 153, it is possible to avoid that, in the event of misalignment, the first output portion 3114 of the first driving member 311 collides with the adjustment assembly 10, which causes unnecessary damages to the parts.

In step S103, the engaging portion 413 is driven by the operating portion 412 to move toward the fixing portion 422 such that the loop structure of the engaging portion 413 passes through the fixing portion 422, the operating portion 412 is then moved away from the engaging portion 413, the fixing portion 422 stops the engaging portion 413 from being disengaged therefrom, and at this point the first driving assembly 31 is fixed relative to the adjustment assembly 10.

In step S104, if there is the second driving assembly, the second driving assembly is fixed to the adjustment assembly 10.

In step S200, the output portion of the driving assembly 30 is adaptively interfaced with the input portion of the adjustment assembly 10.

In actual operation, step S200 includes the following steps.

The output portion includes a first output portion 3114, a second output portion 3122, a third output portion 3134 and/or a fourth output portion, and the input portion includes a first manipulating portion 1111, a second manipulating portion 1211, a third manipulating portion 1311 and/or a fourth manipulating portion 1411.

In step S201, the limiting block 3196 is rotated to separate the limiting block 3196 from the positioning block 3197, the return member 3198 causes the connecting block 3191 to return, and at this point the first output portion 3114 is adjustably interfaced with the first manipulating portion 1111.

In step S202, the second driving member 312 and the third driving member 313 are moved along the second path such that the second output portion 3122 and the third output portion 3134 are substantially aligned to the second manipulating portion 1211 and the third manipulating portion 1311. Further, the second driving member 312 and the third driving member 313 are moved along the first path such that the second output portion 3122 and the third output portion 3134 are interfaced with the second manipulating portion 1211 and the third manipulating portion 1311.

In step S203, if there is the fourth output portion, the fourth output portion is interfaced with the fourth manipulating portion 1411.

In step S300, the irradiated body is placed on the carrier 1, the driving assembly 30 is controlled to drive the adjustment assembly 10 to adjust the carrier 1 to the preset spatial position.

In actual operation, step S300 includes the following steps.

In step S301, simulated positioning is carried out by controlling at least one of the first driving member 311, the second driving member 312, the third driving member 313 and the fourth driving member in a wired or wireless manner such that at least one of the first manipulating portion 1111, the second manipulating portion 1211, the third manipulating portion 1311 and the fourth manipulating portion 1411 is rotated to cause the irradiated body to reach the preset position.

It may be understood that, in the above method, the order in which the first driving member 311, the second driving member 312, the third driving member 313 and the fourth driving member are manipulated is not limited.

In step S400, the driving assembly 30 is disengaged from the adjustment assembly 10 by means of the mounting assembly 40. In particular, the operating portion 412 is driven toward the fixing portion 422, the engaging portion 413 is separated from the fixing portion 422, and at this point the first driving assembly 31 and/or the second driving assembly can be disengaged from the adjustment assembly 10.

The adjustment assembly 10 and the driving assembly 30 are moved to the treatment room, the above method steps S100, S200, S300, S400 are repeated to adjust the adjustment assembly 10 to the corresponding position, the driving assembly 30 is then moved out of the treatment room, and finally the irradiated body is irradiated with a beam. It may be understood that the adjustment device may be operated only in the preparation room or only in the treatment room, with no limitation on the place of operation.

It should be understood that although the method numbers involved in the embodiments as described above are shown sequentially, the method numbers are not necessarily performed sequentially in the order. Unless explicitly stated herein, the order, in which these method numbers are performed, is not strictly limited, and these method numbers may be performed in other orders. Moreover, at least some of the numbers involved in the embodiments as described above may include a plurality of method numbers or a plurality of stages. These method numbers or stages are not necessarily performed at the same time and instead they may be performed at different times. These method numbers or stages are not necessarily performed in a sequential order, and instead they may be performed in turn or alternately with other numbers or at least some of steps or stages of the other numbers.

The technical features of the foregoing embodiments may be combined arbitrarily. To make the description concise, not all possible combinations of the technical features in the foregoing embodiments are described one by one. However, the combinations of these technical features shall be considered as falling within the scope of this specification provided that no conflict exists.

The embodiments described above merely illustrate several implementations of the present application and are described relatively specifically and in detail, but should not be construed as limiting the patent scope of the present application. It should be noted that several variations and improvements may also be made by those of ordinary skill in the art without departing from the concept of the present application, and those modifications and improvements shall all fall within the scope of protection of the present application. Therefore, the scope of protection of the present patent application shall be subject to the appended claims.

## Claims

1. An adjustment device for radiotherapy, **characterized by** comprising:
a first adjustment unit configured to adjust an object to move along a first degree of freedom; and
a second adjustment unit linked to the first adjustment unit and configured to adjust the object to move along a second degree of freedom, wherein the first adjustment unit, when working, is capable of driving the second adjustment unit to move along the first degree of freedom.

2. The adjustment device for radiotherapy according to claim 1, **characterized in that** the first adjustment unit comprises a first adjustment member, a first transmission member, and a first support member, the first adjustment member being configured to be connected to and drive the first transmission member to move, the first support member being configured to support the first adjustment member, and the first transmission member being connected to the second adjustment unit.

3. The adjustment device for radiotherapy according to claim 1, **characterized by** further comprising a third adjustment unit, the third adjustment unit being configured to adjust the object to move along a third degree of freedom and being linked to the second adjustment unit, and the second adjustment unit, when working, being capable of driving the third adjustment unit to move along the second degree of freedom.

4. The adjustment device for radiotherapy according to claim 3, **characterized in that** the second adjustment unit comprises a second adjustment member, a second transmission member, a fixed member, and a second support member, the second adjustment member being configured to be connected to and drive the second transmission member to move, the second transmission member being connected to the fixed member, the second support member being configured to support the fixed member, and the second transmission member being connected to the third adjustment unit.

5. The adjustment device for radiotherapy according to claim 3, **characterized in that** the third adjustment unit comprises a third adjustment member, a swinging member, a third support member, and a first swinging guiding member, the third adjustment member being configured to be connected to and drive the swinging member to move, the third support member being configured to support the third adjustment member, the first swinging guiding member being disposed on the third support member, and the first swinging guiding member being configured to guide a movement path of the swinging member.

6. The adjustment device for radiotherapy according to claim 5, **characterized in that** the swinging member is movable relative to the third adjustment member, and the first swinging guiding member is configured as an arc such that the swinging member is adjustable relative to a direction of movement of the third adjustment member along the first swinging guiding member.

7. The adjustment device for radiotherapy according to claim 6, **characterized in that** a limiting portion is provided on the third adjustment member, an adjustment location for movement of the limiting portion is provided on the swinging member, and when the third adjustment member drives the swinging member to move, the swinging member swings relative to the first swinging guiding member, and the limiting portion moves within the adjustment location.

8. The adjustment device for radiotherapy according to claim 7, **characterized in that** the third adjustment unit further comprises a rotating member, the object is rotatably connected to the third support member by means of the rotating member, and when the swinging member drives the object to move, the object rotates about an axis of the rotating member.

9. The adjustment device for radiotherapy according to claim 8, **characterized in that** the third adjustment unit further comprises a second swinging guiding member for guiding the movement of the object, the second swinging guiding member being configured as an arc, and the second swinging guiding member being disposed concentrically with the first swinging guiding member.

10. The adjustment device for radiotherapy according to claim 3, **characterized by** further comprising a fourth adjustment unit configured to adjust the object to move at least along a fourth degree of freedom, the fourth adjustment unit being linked to the third adjustment unit, and the third adjustment unit, when working, being capable of driving the fourth adjustment unit to move along the third degree of freedom.

11. The adjustment device for radiotherapy according to claim 10, **characterized in that** the fourth adjustment unit comprises a fourth adjustment member, a fourth support member, a third transmission member, and a lifting member, the fourth support member being configured to support the fourth adjustment member, the fourth adjustment member being connected to the third transmission member, the third transmission member being connected to the lifting member, the fourth adjustment member driving the third transmission member to move in a first direction, and the third transmission member driving the lifting member to move in a second direction perpendicular to the first direction.

12. The adjustment device for radiotherapy according to claim 11, **characterized in that** the fourth adjustment unit further comprises a lifting guiding member configured to guide a movement of the lifting member.

13. The adjustment device for radiotherapy according to claim 1, **characterized in that** a casing is provided outside at least the first adjustment unit and the second adjustment unit, the casing comprising at least a first housing and a second housing, and when the first adjustment unit and/or the second adjustment unit move, the first housing and the second housing adjust shapes in a direction of the first degree of freedom and/or the second degree of freedom with the movement of the first adjustment unit and/or the second adjustment unit.

14. An adjustment method for an adjustment device for radiotherapy comprising a first adjustment unit, a second adjustment unit, and a third adjustment unit, **characterized by** comprising:
driving the first adjustment unit to move along a first degree of freedom, the first adjustment unit driving the second adjustment unit to move simultaneously along the first degree of freedom; and/or
driving the second adjustment unit to move along a second degree of freedom, the second adjustment unit driving the third adjustment unit to move simultaneously along the second degree of freedom.

15. An carrying system for an adjustment device for radiotherapy, **characterized by** comprising:
a neutron generation device configured to generate a neutron beam for irradiation treatment of an irradiated body;
a carrier configured to carry the irradiated body;
an adjustment assembly configured to adjust a spatial position of the carrier and connected to the carrier, the adjustment assembly comprising at least a first adjustment unit and a second adjustment unit; wherein
the first adjustment unit is configured to adjust the carrier to move along a first degree of freedom,
the second adjustment unit is configured to adjust the carrier to move along a second degree of freedom, and
the first adjustment unit moving along the first degree of freedom drives the second adjustment unit to move simultaneously along the first degree of freedom.
